# EUROPEAN PATENT APPLICATION

(11) **EP 3 309 147 A1**
(43) Date of publication of application: **18.04.2018**
(21) Application number: 16193554.9
(22) Date of filing: 12.10.2016
(51) Int. Cl.: C07C 409/16, H01M 10/0569

(54) **FLUORINATED PEROXIDES, THEIR USE AS ELECTROLYTE COMPONENT AND PROCESS FOR THEIR PREPARATION**

(71) Applicant: Solvay SA, 1120 Bruxelles (BE)
(72) Inventor: FABRE, Jean, 30457 Wettbergen (DE); BUISINE, Olivier, 69230 Saint Genis-Laval (FR); PERNICE, Holger, 28790 SCHWANEWEDE (DE)
(74) Representative: Menville, Laure

(57) **Abstract**

The present invention concerns compounds of formula R1-O-O-R2 (Formula(I)) wherein R1 and R2 are perfluorinated or partially fluorinated branched or linear alkyl, their use as electrolyte component and processes for their preparation.

## Description

The present invention relates to the technical field of batteries. More specifically it concerns perfluorinated or partially fluorinated peroxides, their use as electrolyte component and a process for the preparation of such compounds.

### Background of the invention

In the field of battery, it is advantageous to have a liquid electrolyte component which is stable at high temperature and which can be preferably compatible with high voltage batteries.

The development of new compounds which are more stable, and could be used as electrolyte component is therefore of particular interest.

Fluorinated peroxides are mainly known as gas. For example CF₃-O-O-CF₃ has been described in WO 2014/096414 as alternative to SF₆ and N₂ as dielectric insulating gas. However the use of such fluorinated peroxides in the battery field has never been suggested.

The object of the present invention is to provide new fluorinated or partially fluorinated compounds which are useful as electrolyte component. This object and other objects are achieved by this invention. Advantageously the compounds of the present invention are liquid at standard temperature and pressure. Additionally the polarity of the compounds of the present invention is tunable in order to adjust their solubility properties. Eventually the compounds of the present invention can also be destroyed at very high voltage in order to contribute to the surface electrolyte interface, and thus improve the cycle life of battery.

### Brief description of the invention

Accordingly a first aspect of the present invention concerns a compound of formula R1-O-O-R2 (Formula (I)) wherein R1 is C(CF₃)₃ and R2 is a perfluorinated or partially fluorinated linear or branched alkyl and wherein R2 is different from R1.

In another aspect, the present invention concerns the use of at least one compound of formula (I) R1-O-O-R2 as electrolyte component wherein R1 and R2 are perfluorinated or partially fluorinated linear or branched alkyl.

In a further aspect, the present invention concerns an electrolyte composition for an electrochemical device comprising at least one compound of formula (I) R1-O-O-R2 wherein R1 and R2 are perfluorinated or partially fluorinated linear or branched alkyl.

In still another aspect, the present invention concerns a battery or a supercapacitor comprising an electrolyte composition comprising at least one compound of formula (I) R1-O-O-R2 wherein R1 and R2 are perfluorinated or partially fluorinated linear or branched alkyl.

In a further aspect the present invention concerns a process for the manufacture of a compound of formula (I) R1-O-O-R2 wherein R1 and R2 are perfluorinated or partially fluorinated linear or branched alkyl using the appropriate R1-OH and the appropriate R2-OF.

In another aspect the present invention concerns a process for the manufacture of a compound of formula (I) R1-O-O-R2 wherein R1=R2 and R1 and R2 are perfluorinated or partially fluorinated linear or branched alkyl using the appropriate R1-OH in the presence of ClF₃.

In another aspect the present invention concerns a process for the manufacture of a compound of formula (I) R1-O-O-R2 wherein R1 is CF₃ and R2 is perfluorinated or partially fluorinated linear or branched alkyl using R2-OF in the presence of CF₂O and, optionally a catalyst.

### Detailed description

In the context of the present invention, the term "comprising" is intended to include the meaning of "consisting of". In the present disclosure, designations in singular are intended to include the plural. The expression "comprised between... and..." should be understood as including the limits.

In the present disclosure "alkyl" refers to a linear or branched saturated hydrocarbon, having from 1 to 5 carbon atoms. Preferred examples of alkyl groups are methyl, ethyl, propyl, isopropyl, butyl, isobutyl and tert-butyl.

The term "perfluorinated alkyl" refers to a linear or branched saturated hydrocarbon, having from 1 to 5 carbon atoms wherein all the hydrogen atoms have been replaced by fluorine. Preferred examples are CF₃, CF₃CF₂, CF₃CF₂CF₂, (CF₃)₂CF, CF₃CF₂CF₂CF₂, CF₃CF₂(CF₃)CF₂, (CF₃)₃C.

The term "partially fluorinated alkyl refers to a linear or branched saturated hydrocarbon, having from 1 to 5 carbon atoms wherein at least one hydrogen atoms has been replaced by fluorine. Preferred examples are CH₂F, CHF₂, CF₃CH₂, CF₃CHF, CF₃CH₂CH₂, CF₃CF₂CH₂, (CF₃)₂CH.

The term "standard temperature and pressure" should be understood as a temperature of around 20-25°C and at a pressure of 1 bar.

A first aspect of the present invention concerns a compound of formula R1-O-O-R2 (Formula (I)) wherein R1 is C(CF₃)₃ and R2 is a perfluorinated or partially fluorinated linear or branched alkyl and wherein R2 is different from R1.

Preferably R2 is selected from the group consisting of perfluorinated or partially fluorinated methyl, ethyl, isopropyl, n-propyl, isobutyl, n-butyl, and t-butyl. Preferably R2 is selected from CF₃, CF₂CF₃, CF(CF₃)₂.

In another aspect R2 is selected from CHF₂, CF₃CHF, CF₃CF₂CHF, (CF₃)₂CH, (CF₃)₂(CH₃)C, (CF₃)(CH₃)₂C.

According to an advantageous aspect of the invention, the compound of formula (I) R1-O-O-R2 is liquid at standard temperature and pressure. Accordingly the compound of formula (I) R1-O-O-R2 may have a boiling point equal to or higher than 25°C, preferably equal to or higher than 40°C, more preferably equal to or higher than 50°C, and still more preferably equal to or higher than 70°C. The compound of formula (I) R1-O-O-R2 may have a boiling point equal to or less than 200°C, preferably equal to or less than 190°C, more preferably equal to or less than 185°C.

Advantageously, one subject-matter of the present invention is a compound selected from the groups consisting of (CF₃)₃C-O-O-CF₃, (CF₃)₃C-O-O-CF₂CF₃, (CF₃)₃C-O-O-CF(CF₃)₂, (CF₃)₃C-O-O-CHF₂, (CF₃)₃C-O-O-CHFCF₃, (CF₃)₃C-O-O-CHFCF₂CF₃,(CF₃)₃C-O-O-CH(CF₃)₂ and mixtures thereof.

According to a preferred embodiment, the compound of formula (I) R1-O-O-R2 for use according to the invention may have R1 is C(CF₃)₃. Therefore, the present invention concerns the use of at least one compound of formula (I) R1-O-O-R2 as electrolyte component, wherein R1 is C(CF₃)₃ and R2 is perfluorinated or partially fluorinated linear or branched alkyl.

Preferably R2 can be selected from the group consisting of perfluorinated or partially fluorinated methyl, ethyl, isopropyl, n-propyl, isobutyl, n-butyl, and tert-butyl. According to one aspect of the invention, R1 and R2 may be the same. According to another aspect, R1 and R2 may be different. R2 can independently be selected from the group consisting of CF₃, CF₂CF₃, CF(CF₃)₂ and C(CF₃)₃. In another aspect, R2 can independently be selected from the group consisting of CHF₂, CF₃CHF, CF₃CF₂CHF and (CF₃)₂CH.

Advantageously, the compounds of formula (I) R1-O-O-R2 wherein R1 is C(CF₃)₃ and R2 is perfluorinated or partially fluorinated linear or branched alkyl used in the present invention as electrolyte component is liquid at standard temperature and pressure. Accordingly the compound of formula (I) R1-O-O-R2 wherein R1 is C(CF₃)₃ and R2 is perfluorinated or partially fluorinated linear or branched alkyl may have a boiling point equal to or higher than 25°C, preferably equal to or higher than 40°C, more preferably equal to or higher than 50°C, and still more preferably equal to or higher than 70°C. Said compounds of formula (I) R1-O-O-R2 wherein R1 is C(CF₃)₃ and R2 is perfluorinated or partially fluorinated linear or branched alkyl may have a boiling point equal to or less than 200°C, preferably equal to or less than 190°C, more preferably equal to or less than 185°C.

Specifically, one subject-matter of the present invention is the use of at least one compound selected from the group consisting of (CF₃)₃-O-O-C(CF₃)₃, CF₃-O-O-C(CF₃)₃, CF₃CF₂-O-O-C(CF₃)₃, CF(CF₃)₂-O-O-C(CF₃)₃, (CF₃)₃C-O-O-CHF₂, (CF₃)₃C-O-O-CHFCF₃, (CF₃)₃C-O-O-CHFCF₂CF₃, (CF₃)₃C-O-O-CH(CF₃)₂ and mixture thereof, as electrolyte component.

In another embodiment the present invention concerns the use of at least one compound of formula (I) R1-O-O-R2 as electrolyte component, wherein R1 and R2 are perfluorinated or partially fluorinated linear or branched alkyl.

Preferably R1 and R2 can independently be selected from the group consisting of perfluorinated or partially fluorinated methyl, ethyl, isopropyl, n-propyl, isobutyl, n-butyl, and tert-butyl. According to one aspect of the invention, R1 and R2 may be the same. According to another aspect, R1 and R2 may be different. R1 and R2 can independently be selected from the group consisting of CF₃, CF₂CF₃, CF(CF₃)₂ and C(CF₃)₃. In another aspect, R1 and R2 can independently be selected from the group consisting of CHF₂, CF₃CHF, CF₃CF₂CHF and (CF₃)₂CH.

Advantageously the compounds of formula (I) R1-O-O-R2 wherein R1 and R2 are perfluorinated or partially fluorinated linear or branched alkyl used in the present invention as electrolyte component is liquid at standard temperature and pressure. Accordingly the compound of formula (I) R1-O-O-R2 wherein R1 and R2 are perfluorinated or partially fluorinated linear or branched alkyl may have a boiling point equal to or higher than 25°C, preferably equal to or higher than 40°C, more preferably equal to or higher than 50°C, and still more preferably equal to or higher than 70°C. Said compounds of formula (I) R1-O-O-R2 wherein R1 and R2 are perfluorinated or partially fluorinated linear or branched alkyl may have a boiling point equal to or less than 200°C, preferably equal to or less than 190°C, more preferably equal to or less than 185°C.

Specifically one subject-matter of the present invention is the use of at least one compound selected from the group consisting of CF₃CF₂-O-O-CF₂CF₃, (CF₃)₂CF-O-O-CF(CF₃)₂, (CF₃)₃-O-O-C(CF₃)₃, CF₃-O-O-C(CF₃)₃, CF₃CF₂-O-O-C(CF₃)₃, CF₃CF₂-O-O-CF(CF₃)₂, CF(CF₃)₂-O-O-C(CF₃)₃, (CF₃)₃C-O-O-CHF₂, (CF₃)₃C-O-O-CHFCF₃, (CF₃)₃C-O-O-CHFCF₂CF₃, (CF₃)₃C-O-O-CH(CF₃)₂ and mixtures thereof, as electrolyte component.

Preferably the compounds used in the present invention are used as electrolyte component in an electrochemical device, and more particularly in electronic displays or in energy storing and releasing devices. The compounds of formula (I) R1-O-O-R2 wherein R1 and R2 are perfluorinated or partially fluorinated linear or branched alkyl may be used as electrolyte component in one of the following electrochemical devices:
- Electrochromic device: car or house windows, visors, eyeglasses,
- Electrochromic flat screens: televisions, tablets, smartphones, connected devices,
- Secondary lithium batteries, lithium-sulfur batteries, lithium-air batteries, sodium batteries,
- High voltage batteries,
- Supercapacitors, in particular double-layer supercapacitors using an electrolyte,
- Energy generator, such as solar panels or organic type (OPV).
Preferably the electrochemical device may be a lithium-ion battery, a lithium-sulfur battery or a supercapacitor.

The compounds of formula (I) R1-O-O-R2 wherein R1 and R2 are perfluorinated or partially fluorinated linear or branched alkyl may be used as additive in an electrolyte composition. Alternatively, the compounds of formula (I) R1-O-O-R2 wherein R1 and R2 are perfluorinated or partially fluorinated linear or branched alkyl may be used as solvent or co-solvent in an electrolyte composition. Preferably the compounds of formula (I) R1-O-O-R2 wherein R1 and R2 are perfluorinated or partially fluorinated linear or branched alkyl may be used as additive, solvent or co-solvent for lithium-ion batteries, lithium-sulfur batteries or supercapacitors.

In another embodiment, the present invention concerns an electrolyte composition for an electrochemical device comprising at least one compound of formula (I) R1-O-O-R2 wherein R1 and R2 are perfluorinated or partially fluorinated linear or branched alkyl.

The compounds of formula (I) R1-O-O-R2 wherein R1 and R2 are perfluorinated or partially fluorinated linear or branched alkyl are advantageously used in electrolyte compositions together with at least one suitable solvent known to the person skilled in the art of lithium-ion batteries, lithium-sulfur batteries or supercapacitors. For example, organic carbonates, but also lactones, formamides, pyrrolidinones, oxazolidinones, nitroalkanes, N,N-substituted urethanes, sulfolanes, dialkyl sulfoxides, dialkyl sulfites, acetates, nitriles, acetamides, glycol ethers, dioxolanes, dialkyloxyethanes, trifluoroacetamides are very suitable as solvents.

Preferably the electrolyte composition of the present invention comprises at least one solvent, preferably a cyclic or non-cyclic carbonate, more preferably selected from ethylene carbonate, dimethyl carbonate, triglyme or mixture thereof.

The compounds of formula (I) R1-O-O-R2 wherein R1 and R2 are perfluorinated or partially fluorinated linear or branched alkyl may advantageously be used as a solvent, a co-solvent, or an additive in a concentration equal to or more than 1 % by weight, preferably equal to or more than 3% by weight, more preferably equal to or more than 4% by weight, most preferably around 5% by weight relative to the total weight of the electrolyte composition. The compounds of formula (I) R1-O-O-R2 wherein R1 and R2 are perfluorinated or partially fluorinated linear or branched alkyl may advantageously be used as a solvent, a co-solvent, or an additive in a concentration equal to or less than 20% by weight, preferably equal to or less than 10% by weight, more preferably equal to or less than 6% by weight relative to the total weight of the electrolyte composition.

Accordingly another aspect of the present invention concerns the use of a compound of formula (I) R1-O-O-R2 wherein R1 and R2 are perfluorinated or partially fluorinated linear or branched alkyl in an electrolyte composition wherein the concentration of said compound is equal to or more than 1% by weight, preferably equal to or more than 3% by weight, more preferably equal to or more than 4% by weight, most preferably around 5% by weight relative to the total weight of the electrolyte composition. Another aspect of the present invention concerns the use of a compound of formula (I) R1-O-O-R2 wherein R1 and R2 are perfluorinated or partially fluorinated linear or branched alkyl in an electrolyte composition wherein the concentration of said compound is equal to or less than 20% by weight, preferably equal to or less than 10% by weight, more preferably equal to or less than 6% by weight relative to the total weight of the electrolyte composition.

In another aspect, the compounds of formula (I) R1-O-O-R2 wherein R1 and R2 are perfluorinated or partially fluorinated linear or branched alkyl are advantageously used in electrolyte compositions together with at least one electrolyte salt known to the person skilled in the art of lithium-ion batteries, lithium-sulfur batteries or supercapacitors. Such salts have the general formula MₐA_{b}, wherein M represents a metal cation, A represents an anion, a represents 1, 2, 3 or 4, b represents 1, 2 ,3 or 4, and the overall charge of the salt MₐA_{b} is 0. M is preferably selected from Li⁺ and NR₄⁺. Preferred anions are PF₆⁻, PO₂F₂⁻, AsF₆⁻, BF₄⁻, ClO₄⁻, N(CF₃SO₂)²⁻, N(FSO₂)²⁻ and N(i-C₃F₇SO₂)²⁻. Preferably the electrolyte composition of the present invention comprises at least one lithium salt, preferably selected from LiTFSI or LiPF6. The concentration of the electrolyte salt is preferably between 0.8 and 1.2 molar, more preferably 1.0 molar. The compound of formula (I) wherein R1 and R2 are perfluorinated or partially fluorinated linear or branched alkyl can be introduced into the electrolyte composition separately or in the form of a mixture with other compounds, for example as a mixture with one or more solvents used in the electrolyte composition or together with the electrolyte salt or together with other additives.

Advantageously the compounds of formula (I) R1-O-O-R2 wherein R1 and R2 are perfluorinated or partially fluorinated linear or branched alkyl display a good compatibility with an electrolyte composition.

In another embodiment, the present invention concerns a battery, preferably a high-voltage battery, or a supercapacitor comprising an electrolyte composition comprising at least one compound of formula (I) R1-O-O-R2 wherein R1 and R2 are perfluorinated or partially fluorinated linear or branched alkyl. Batteries according to the invention may be low temperature batteries (working typically between -40°C and 0°C), or batteries working higher temperatures, between 0° and the ambient temperature, or at a temperature equal to or higher than 25°C, preferably equal to or higher than 40°C, more preferably equal to or higher than 50°C, and still more preferably equal to or higher than 70°C.

Lithium-ion batteries comprise an anode, preferably an anode made from carbon on a copper foil, a cathode, preferably a cathode made from lithium metal oxides on an aluminium foil, a separator, preferably a separator made from an insulating polymer, and a solvent composition or an electrolyte composition as described above. The foils used for anode and cathode are also called current collectors.

In another aspect, the present invention relates to a process for the manufacture of a compound of formula (I) R1-O-O-R2 wherein R1 and R2 are perfluorinated or partially fluorinated linear or branched alkyl using the appropriate R1OH and the appropriate R2OF. Said process may comprise the step consisting of reacting a compound of formula R1OH and a compound of formula R2OF together to obtain a compound of formula (I) R1-O-O-R2, wherein R1 and R2 are perfluorinated or partially fluorinated linear or branched alkyl.

In an aspect of this embodiment R1 and R2 may be the same. In another aspect R1 and R2 may be different.

The reaction of this embodiment can be carried out in a static or continuous-flow reactor.

The reaction of this embodiment may be carried out at a temperature equal to or more than -196°C, preferably equal to or more than -100°C, more preferably equal to or more than - 78°C. The reaction of this embodiment may be carried out at a temperature equal to or less than 180°C, preferably equal to or less than 100°C, more preferably equal to or less than 50°C. Specifically the reaction of this embodiment may be carried out at a temperature of about 0°C.

In another aspect, the present invention relates to a process for the manufacture of a compound of formula (I) R1-O-O-R2 wherein R1 is C(CF₃)₃ and R2 is perfluorinated or partially fluorinated linear or branched alkyl using the appropriate R1OH and the appropriate R2OF. Said process may comprise the step consisting of reacting a compound of formula R1OH and a compound of formula R2OF together to obtain a compound of formula (I) R1-O-O-R2, wherein R1 is C(CF₃)₃ and R2 is perfluorinated or partially fluorinated linear or branched alkyl.

In an aspect of this embodiment R1 and R2 may be the same. In another aspect R1 and R2 may be different.

The reaction of this embodiment can be carried out in a static or continuous-flow reactor.

The reaction of this embodiment may be carried out at a temperature equal to or more than -196°C, preferably equal to or more than -100°C, more preferably equal to or more than-78°C. The reaction of this embodiment may be carried out at a temperature equal to or less than 180°C, preferably equal to or less than 100°C, more preferably equal to or less than 50°C. Specifically the reaction of this embodiment may be carried out at a temperature of about 0°C.

In another aspect, the present invention relates to a process for the manufacture of a compound of formula (I) R1-O-O-R2 wherein R1 and R2 are perfluorinated or partially fluorinated linear or branched alkyl having wherein R1 = R2 using R1OH in the presence of ClF₃ as described in FR2050173.

Preferably the reaction may be performed using 2 equivalents of R1OH.

Preferably R1 and R2 are different from CF₃ or (CF₃)₃.

The reaction of this embodiment can be carried out in a static or continuous-flow reactor.

The reaction of this embodiment may be carried out at a temperature equal to or more than -196°C, preferably equal to or more than -100°C, more preferably equal to or more than - 78°C. The reaction of this embodiment may be carried out at a temperature equal to or less than 30°C, preferably equal to or less than 15°C, more preferably equal to or less than 0°C. Specifically the reaction of this embodiment may be carried out at a temperature of -78°C.

In another aspect, the present invention relates to a process for the manufacture of a compound of formula (I) R1-O-O-R2 wherein R1 is CF₃ and R2 is perfluorinated or partially fluorinated linear or branched alkyl using the appropriate hypofluorite R2OF in the presence of CF₂O as described in EP3078657.

In one alternative the carbonyl fluoride is prepared in situ in a flow reactor, preferably prepared in situ from F₂ and CO. The mixture of F₂ and CO is suitably ignited to prepare COF₂, for example by means of an electric spark.

Preferably R2 is different from R1.

Optionally the reaction is carried out in the presence of a catalyst. Preferably the catalyst is selected from the group consisting of AgF, CuF, CuF₂, BaF₂, CsF, NiF₂, preferably AgF.

The reaction of this embodiment can be carried out in a static or continuous-flow reactor. Preferably, the reactor is made of a material suitable for reactions involving molecular fluorine, for example made of Monel® 400 or nickel. Also preferably, the reaction is carried out in a continuous-flow reactor.

The reaction of this embodiment may be carried out at a temperature equal to or more than -20°C, preferably equal to or more than 0°C, more preferably equal to or more than 20°C. The reaction of this embodiment may be carried out at a temperature equal to or less than 180°C, preferably equal to or less than 140°C, more preferably equal to or less than 50°C. Specifically the reaction of this embodiment may be carried out at a temperature of 25°C.

Should the disclosure of any patents, patent applications and publications, which are incorporated herein by reference, conflicts with the description of the present invention to the extent that it may render a term unclear, the present description shall take precedence.

The invention will now be further described in examples, which are given by way of illustration and which are no intended to limit the specification or the claims in any manner.

### Examples

### Example 1: Manufacture of (CF₃)₃COOCF₃

An equimolar mixture of carbonyl fluoride (COF₂) and perfluoro-t-butyl hypofluorite (CF₃)₃COF is allowed to react in a static reactor made from Monel 400 in the presence of silver fluoride AgF at 25°C for 24 hours.

### Example 2: Manufacture of CF₃CF₂OOCF₂CF₃

An equimolar mixture of perfluoro-ethanol (CF₃CF₂OH) and chlorine trifluoride (ClF₃) is allowed to react in a static reactor at -78°C for 12 hours.

### Example 3: Manufacture of (CF₃)₃COOCF₂CF₃

An equimolar mixture of perfluoro-t-butyl hypofluorite (CF₃)₃COF and perfluoro-ethanol (CF₃CF₂OH) is allowed to react in a static reactor at 0°C for 12 hours.

### Example 4: Preparation of electrolyte compositions

Battery grade 1M LiPF₆ in ethylene carbonate/dimethyl carbonate (1/2, v/v) is used as the bas electrolyte solution under dry room atmosphere. The following electrolyte compositions were used:
Example A (comparative): No additive
Example B (Invention): 0.1% (CF₃)₃COOC(CF₃)₃
Example C (Invention): 0.5% (CF₃)₃COOC(CF₃)₃

It appears that the perfluoroperoxide (CF₃)₃COOC(CF₃)₃ displays good compatibility with the electrolyte composition at various temperature and pressure.

## Claims

1. Compound of formula R1-O-O-R2 (Formula (I)) wherein R1 is C(CF₃)₃ and R2 is a perfluorinated or partially fluorinated linear or branched alkyl and wherein R2 is different from R1.

2. Compound according to claim 1, wherein said compound is liquid at standard temperature and pressure.

3. Compound according to any one of claims 1 or 2, wherein said compound of formula (I) has a boiling point equal to or higher than 25°C, preferably equal to or higher than 40°C, more preferably equal to or higher than 50°C and still more preferably equal to or higher than 70°C.

4. Compound according to any one of claims 1 to 3, wherein said compound is selected from the groups consisting of (CF₃)₃C-O-O-CF₃, (CF₃)₃C-O-O-CF₂CF₃, (CF₃)₃C-O-O-CF(CF₃)₂, (CF₃)₃C-O-O-CHF₂, (CF₃)₃C-O-O-CHFCF₃, (CF₃)₃C-O-O-CHFCF₂CF₃,(CF₃)₃C-O-O-CH(CF₃)₂ and mixtures thereof.

5. Use of at least one compound of formula (I) R1-O-O-R2 as electrolyte component wherein R1 and R2 are perfluorinated or partially fluorinated linear or branched alkyl.

6. Use of at least one compound of formula (I) according to claim 5, wherein said compound of formula (I) is liquid at standard temperature and pressure.

7. Use of at least one compound of formula (I) according to any one of claims 5 to 6, wherein said compound of formula (I) has a boiling point equal to or higher than 25°C, preferably equal to or higher than 40°C, more preferably equal to or higher than 50°C and still more preferably equal to or higher than 70°C.

8. Use of at least one compound of formula (I) according to any one of claims 5 to 7, said compound is selected from the group consisting of (CF₃)₃-O-O-C(CF₃)₃, CF₃-O-O-C(CF₃)₃, CF₃CF₂-O-O-C(CF₃)₃, CF(CF₃)₂-O-O-C(CF₃)₃, (CF₃)₃C-O-O-CHF₂, (CF₃)₃C-O-O-CHFCF₃, (CF₃)₃C-O-O-CHFCF₂CF₃, (CF₃)₃C-O-O-CH(CF₃)₂ and mixtures thereof.

9. Use of at least one compound of formula (I) according to any one of claims 5 to 8 as additive in an electrolyte composition, or as a solvent or a co-solvent in an electrolyte composition.

10. An electrolyte composition for an electrochemical device comprising at least one compound of formula (I) R1-O-O-R2 wherein R1 and R2 are perfluorinated or partially fluorinated linear or branched alkyl, preferably selected from a lithium-ion battery, lithium-sulfur battery or supercapacitor.

11. A battery or a supercapacitor comprising an electrolyte composition as defined in claim 10.

12. Process for the manufacture of a compound of formula (I) R1-O-O-R2 wherein R1 and R2 are perfluorinated or partially fluorinated linear or branched alkyl using the appropriate R1OH and the appropriate R2OF.

13. Process for the manufacture of a compound of formula (I) R1-O-O-R2 wherein R1 and R2 are perfluorinated or partially fluorinated linear or branched alkyl wherein R1=R2 using the appropriate R1OH in the presence of ClF₃.

14. Process for the manufacture of a compound of formula (I) R1-O-O-R2 wherein R1 is CF₃ and R2 is perfluorinated or partially fluorinated linear or branched alkyl using the appropriate hypofluorite R2OF in the presence of CF₂O and optionally a catalyst.

15. Process according to claim 14, wherein the catalyst is selected from the group consisting of AgF, CuF, CuF₂, BaF₂, CsF, NiF₂, preferably AgF.
